# EUROPÄISCHE PATENTSCHRIFT

(11) **EP 0 405 197 B1**
(45) Veröffentlichungstag und Bekanntmachung des Hinweises auf die Patenterteilung: **22.09.1993**
(21) Anmeldenummer: 90110760.7
(22) Anmeldetag: 07.06.1990
(51) Int. Cl.: C12P 7/24

(54) **Verfahren zur Herstellung von natürlichem Vanillin**
Process for production of natural vanillin
Procédé de production de vanilline naturelle

(30) Priorität: 20.06.1989 DE 3920039
(43) Veröffentlichungstag der Anmeldung: 02.01.1991
(73) Patentinhaber: HAARMANN & REIMER GMBH, D-37601 Holzminden (DE)
(72) Erfinder: Rabenhorst, Jürgen, Prof.Dr., D-3454 Bevern (DE); Hopp, Rudolf, Dr., D-3450 Holzminden (DE)
(74) Vertreter: Müller, Gerhard, Dr.

(56) Entgegenhaltungen:
- DE-A- 3 604 874
- AGRICULTURAL AND BIOLOGICAL CHEMISTRY, Band 41, Juni 1977, Tokio K. TADASA "Degradation of Eugenol by a Microorganism" Seiten 925-929
- AGRICULTURAL AND BIOLOGICAL CHEMISTRY, Band 47, September 1983, Tokio K. TADASA et al. "Initial Steps of Eugenol Degradation Pathway of a Microorganism" Seiten 2639-2640
- BIO ENGINEERING, 4. Jahr- gang, Nr. 3, September 1988 GrUfelfing, BRD K. KIESLICH "Mikrobiolo- gische Umwandlungen" Seiten 116-117

## Beschreibung

Die Erfindung betrifft ein Verfahren zur Herstellung von natürlichem Vanillin durch fermentative Oxidation von Eugenol und/oder Isoeugenol.

Vanillin ist ein wichtiger, in der Nahrungs- und Genußmittel-Industrie viel verwendeter Aromastoff. Bislang wird es hauptsächlich aus dem Lignin der Sulfitablaugen, gelegentlich auch durch Oxidation von Eugenol oder Isoeugenol auf chemischem Wege hergestellt. Das auf diese Weise erhaltene Vanillin hat jedoch den Nachteil, daß es im lebensmittelrechtlichen Sinne kein Naturstoff ist und deshalb auch nicht als natürlicher Aromastoff bezeichnet werden darf.

Der natürliche Aromastoff Vanillin ist bislang nur durch Extraktion von Vanilleschoten erhältlich; das auf diese Weise erhaltene natürliche Vanillin ist jedoch für eine Anwendung in industriellem Maßstab viel zu kostspielig.

Aus Agric. Biol. Chem. 41, 925-929 (1977) und 74, 2639-2640 (1983) ist bekannt, daß Eugenol beim mikrobiellen Abbau durch Bakterien der Gattung Corynebacterium sp. und Pseudomonas sp. neben Ferulasäure, Coniferylalkohol, Coniferylaldehyd und Vanillinsäure auch Vanillin liefert. Dieser Abbau ist jedoch für die Herstellung von Vanillin aus Eugenol nicht verwertbar, weil die Mikroorganismen als solche nicht zur Verfügung stehen und außerdem das Vanillin in einem Stoffgemisch anfällt, aus dem es nur mit erheblichem Aufwand in reiner Form isolierbar ist.

Für Isoeugenol wurden bislang nur Pilze gefunden, die in der Lage sind, Isoeugenol zu Vanillin zu oxidieren. Auch bei dieser mikrobiellen Umsetzung entsteht Vanillin im Gemisch mit anderen Verbindungen, weil die Oxidation weiter zu Vanillinsäure führt und außerdem unerwünschte Dimerisierungen des Isoeugenols auftreten (siehe Bioflavor 87, Ed. : P. Schreier, 399-413 (1988); Bio Engineering 4 (3) 116-117 (1988)). Dazu kommen die bekannten Nachteile bei der Fermentation mit mycelbildenden Pilzen, wie langsameres Wachstum, Inhomogenitäten im Medium usw.

Auf der Suche nach einem neuen, praktisch anwendbaren Herstellungsverfahren für natürliches Vanillin wurde jetzt gefunden, daß dieses auf wirtschaftliche Weise mit Hilfe bestimmter, gut zugänglicher Mikroorganismen durch mikrobielle Oxidation von Eugenol und/oder Isoeugenol in guten Ausbeuten erhalten wird.

Überraschenderweise wurde gefunden, daß diese bestimmten Mikroorganismen nicht nur Eugenol sondern auch Isoeugenol in Vanillin überführen.

Die Erfindung betrifft daher ein Verfahren zur Herstellung von natürlichem Vanillin, das dadurch gekennzeichnet ist, daß man Eugenol und/oder Isoeugenol mikrobiell unter Verwendung von Mikroorganismen der Gattungen Serratia, Klebsiella oder Enterobacter, insbesondere von Stämmen von Serratia ficaria, Serratia fonticola, Serratia liquefaciens, Serratia marcescens, Serratia marinorubra, Serratia odorifera, Serratia plymuthica, Serratia rubidaea, Enterobacter aerogenes, Enterobacter agglomerans, Enterobacter cloacae, Klebsiella aerogenes, Klebsiella pneumoniae und Klebsiella edwardsii zu Vanillin oxidiert.

Vertreter der erfindungsgemäß zu verwendenden Mikroorganismen der vorstehend genannten Gattungen sind z.B. die bei der deutschen Sammlung für Mikroorganismen, Braunschweig, hinterlegten Stämme mit den Nummern DSM 49, 1608, 1636, 3264, 30053, 30054, 30063, 30121, 30122, 30124, 30125, 20126 und 30127 oder die bei der ATCC, Rockville, USA, hinterlegten Stämme mit den Nummern ATCC 13 882, 13 883, 13 886, 27593, 27614, 29844, 33077 und 33105 oder die bei der NCTC, London, GB, hinterlegten Stämme NCTC 10 912, 11 214 und 12 147, sowie deren Mutanten. Mutanten dieser Stämme erhält man durch spontane oder induzierte Mutation. Die Mutagenese kann z.B. durch UV-Bestrahlung oder mutagene Substanzen bewirkt werden. Herstellung und Selektion solcher Mutanten sind allgemein bekannte Verfahren.

Das erfindungsgemäße Verfahren wird vorzugsweise wie folgt ausgeführt :

Zunächst wird der erfindungsgemäß zu verwendende Mikroorganismus in einem üblichen Kulturmedium in für die Kultivierung von Mikroorganismen üblichen Weise kultiviert. Das Substrat kann zu Beginn der Inkubation, während oder nach Abschluß des Wachstums auf einmal oder über einen längeren Zeitraum verteilt zugegeben werden. Die Menge an Eugenol bzw. Isoeugenol wird dabei vorteilhaft so bemessen, daß die Konzentration der Verbindungen 0,2 bis 100 g/l Kulturbrühe, vorzugsweise 5 bis 50 g/l Kulturbrühe, beträgt. Der Verlauf der Oxidation wird durch Bestimmung des Vanillingehaltes der Kulturbrühe durch Hochdruckflüssigkeitschromatographie verfolgt. Nachdem die optimale Menge an Vanillin entstanden ist, wird dieses durch bekannte physikalische Verfahren wie Extraktion, Destillation oder Chromatographie aus der Kulturbrühe isoliert. Das so erhaltene Roh-Vanillin kann durch Umkristallisieren aus Wasser weiter gereinigt werden.

Die Kultivierung der erfindungsgemäß zu verwendenden Mikroorganismen kann in synthetischen, halbsynthetischen und natürlichen Kulturmedien erfolgen. Diese Kulturmedien enthalten Kohlenstoffquellen, Stickstoffquellen und gegebenenfalls anorganische Salze, Spurenelemente und Vitamine.

Als Kohlenstoffquellen können z.B. Zucker wie D-Glucose, Zuckeralkohole wie Glycerin oder Mannit, organische Säuren wie Zitronensäure oder komplexe Gemische wie Malzextrakt dienen.

Beispiele für geeignete Stickstoffquellen sind anorganische Stickstoffquellen wie Nitrate und Ammoniumsalze und organische Stickstoffquellen wie Hefeextrakt, Sojamehle, Baumwollsaatmehl, Weizengluten und Maisquellwasser.

Als anorganische Salze können Sulfate, Nitrate, Chloride, Carbonate und Phosphate von Natrium, Kalium, Magnesium, Kalzium, Zink und Eisen verwendet werden.

Die Kultivierungstemperatur liegt vorzugsweise im Bereich von 10 bis 45°C, besonders bevorzugt im Bereich von 25 bis 35°C. Der pH-Wert des Kulturmediums beträgt bevorzugt 3 bis 9, insbesondere 3,5 bis 7,5. Die Kultivierung kann entweder in geeigneten Schüttelapparaturen oder in Fermentern mit Rühreinrichtung erfolgen. Bei der Kultivierung ist für eine ausreichende Belüftung Sorge zu tragen. Die Kultivierung kann batchweise, halbkontinuierlich und kontinuierlich durchgeführt werden. Die Kulturdauer bis zur Erreichung einer maximalen Vanillinmenge liegt zwischen 48 und 300 Stunden ab Beginn der Substratzugabe. Um die Mikroorganismen vor der toxischen Wirkung des Eugenols bzw. Isoeugenols zu schützen, kann es vorteilhaft sein, den Kulturmedien Adsorptionsmittel für die Substrate zuzusetzen, z.B. Aktivkohle oder Adsorberharze wie Amberlite XAD-2, Lewatit OC 1062, Lewatit OC 1064, Amberlite XAD-7 und Amberlite XAD-16.

### Beispiel 1

Zehn 500 ml Erlenmeyerkolben werden mit je 100 ml einer Lösung von 10 g Glycerin, 3,125 g Na₂HPO₄, 2,5 g KH₂PO₄, 2,5 g (NH₄)₂SO₄, 0,025 g FeSO₄ x 7 H₂O in 1000 ml Wasser gefüllt und anschließend 20 Min. bei 121°C sterilisiert. Anschließend wird jeder Erlenmeyerkolben mit 0,2 ml einer 1-molaren MgSO₄-Lösung und 0,3 ml einer 0,1-molaren CaCl₂-Lösung versetzt und nach dem Abkühlen mit dem Mikroorganismus Serratia marcescens DSM 30126 beimpft. Die Kulturen werden auf einer rotierenden Schüttelmaschine bei 27° C und 150 Upm inkubiert. Nach 2 Tagen werden die einzelnen Kulturen mit je 2 g Isoeugenol versetzt und weiter bei 27° C rotierend geschüttelt. Nach 10 Tagen wird der Vanillingehalt in den Kulturbrühen mittels HPLC bestimmt. Die Vanillinausbeute beträgt dann 900 mg/l entsprechend einer Ausbeute von etwa 5 % der Theorie, bezogen auf eingesetztes Isoeugenol.

Vergleichbare Ausbeuten werden auch bei Verwendung der Mikroorganismen Serratia ficaria, Serratia fonticola, Serratia liquefaciens, Serratia marinorubra, Serratia odorifera, Serratia plymuthica, Serratia rubidaea, Enterobacter aerogenes, Enterobacter agglomerans, Enterobacter cloacae, Klebsiella aerogenes, Klebsiella pneumoniae und Klebsiella edwardsii erhalten.

### Beispiel 2

Zehn 500 ml Erlenmeyerkolben werden mit je 100 ml einer Lösung von 3 g Fleischextrakt, 5 g Fleischpepton, 1 g Hefeextrakt und 1 g Inositol in 1 l Wasser gefüllt (pH-Wert der Lösung: 7) und 20 Min. bei 121°C sterilisiert. Nach dem Abkühlen werden die Kolben mit dem Mikroorganismus Serratia marcescens DSM 30126 beimpft und auf einer rotierenden Schüttelmaschine bei 27° C und 150 Upm inkubiert. Nach 24 Stunden werden die Kulturen mit je 2 g Eugenol versetzt und weiter bei 27° C rotierend geschüttelt. Nach 13 Tagen beträgt der Vanillingehalt in den Kulturbrühen, bestimmt durch HPLC, 18 mg/l.

Mit den Mikroorganismen der anderen Gattungen, Serratia ficaria, Serratia fonticola, Serratia liquefaciens, Serratia marinorubra, Serratia odorifera, Serratia plymuthica, Serratia rubidaea, Enterobacter aerogenes, Enterobacter agglomerans, Enterobacter cloacae, Serratia marinorubra, Serratia odorifera, Serratia rubidaea, Klebsiella aerogenes, Klebsiella pneumoniae und Klebsiella edwardsii werden annähernd gleiche Ausbeuten erhalten.

### Beispiel 3

10 l des in Beispiel 1 beschriebenen Kulturmediums werden in einem Fermenter sterilisiert und anschließend mit 0,5 l einer 24 Stunden alten Kultur von Serratia marcescens DSM 30126, wie in Beispiel 1 beschrieben, beimpft.

Die Kulturbedingungen sind: 30° C, 600 Upm, 5 l Luft/min. Nach 20 Stunden werden 20 g Isoeugenol zugesetzt und die Kultivierung fortgesetzt. Nach 212 Stunden wird die Fermentation abgebrochen. Der Vanillingehalt in der Kulturbrühe beträgt dann gemäß HPLC 3,8 g/l, entsprechend einer Ausbeute von 20,5 % der Theorie, bezogen auf eingesetztes Isoeugenol.

### Beispiel 4

Eine gemäß Beispiel 1 hergestellte Kulturlösung von Serratia marcescens DSM 30126 wird, wie ebenfalls in Beispiel 1 beschrieben, mit 2 Gew.-% Isoeugenol und 10 Gew.-% Lewatit OC 1062 versetzt und, wie ebenfalls in Beispiel 1 beschrieben, inkubiert. Nach 12 Tagen beträgt der Vanillingehalt der Kulturbrühe gemäß HPLC 2,5 g/l.

Die in den Beispielen 1-4 erhaltenen Fermentationsbrühen werden zur Abtrennung des nicht umgesetzten Eugenols bzw. Isoeugenols bei Normaldruck mit Wasserdampf andestilliert. Nach dem Abkühlen wird die Fermentationsbrühe mit einem mit Wasser nicht mischbaren Lösungsmittel, z.B. Diisopropylether oder Essigester, solange extrahiert, bis in der wäßrigen Phase kein Vanillin mehr nachweisbar ist. Die vereinigten Extrakte werden nach dem Trocknen vom Lösungsmittel befreit. Die Kugelrohrdestillation des Rückstandes liefert ein Rohvanillin eines Reinheitsgrades >95 %. Das so erhaltene Rohvanillin kann durch Umkristallisieren aus Wasser noch weiter gereinigt werden.

## Patentansprüche

1. Verfahren zur Herstellung von natürlichem Vanillin, dadurch gekennzeichnet, daß man Eugenol und/oder Isoeugenol mikrobiell unter Verwendung von Mikroorganismen der Gattungen Serratia, Klebsiella oder Enterobacter zu Vanillin oxidiert.

2. Verfahren gemäß Anspruch 1, dadurch gekennzeichnet, daß man als Mikroorganismen der Gattungen Serratia, Klebsiella oder Enterobacter Stämme von Serratia ficaria, Serratia fonticola, Serratia liquefaciens, Serratia marcescens, Serratia marinorubra, Serratia odorifera, Serratia plymuthica, Serratia rubidaea, Enterobacter aerogenes, Enterobacter agglomerans oder Enterobacter cloacae, Klebsiella aerogenes, Klebsiella pneumoniae oder Klebsiella edwardsii verwendet.

3. Verfahren gemäß Anspruch 1, dadurch gekennzeichnet, daß man die mikrobielle Oxidation in üblichen Kulturmedien vornimmt.

4. Verfahren gemäß Anspruch 1, 2 oder 3, dadurch gekennzeichnet, daß man dem Kulturmedium Absorptionsmittel wie Aktivkohle oder Adsorberharze zusetzt.

5. Verfahren nach einem der Ansprüche 1, 2, 3 oder 4, dadurch gekennzeichnet, daß man die Inkubation bei Temperaturen von 10 bis 45° C vornimmt.

## Claims

1. Process for the preparation of natural vanillin, characterised in that eugenol and/or isoeugenol are oxidised microbially using microorganisms of the genera Serratia, Klebsiella or Enterobacter to give vanillin.

2. Process according to Claim 1, characterised in that the microorganisms used, of the genera Serratia, Klebsiella or Enterobacter, are strains of Serratia ficaria, Serratia fonticola, Serratia liquefaciens, Serratia marcescens, Serratia marinorubra, Serratia odorifera, Serratia plymuthica, Serratia rubidaea, Enterobacter aerogenes, Enterobacter agglomerans or Enterobacter cloacae, Klebsiella aerogenes, Klebsiella pneumoniae or Klebsiella edwardsii.

3. Process according to Claim 1, characterised in that the microbial oxidation is carried out in conventional culture media.

4. Process according to Claim 1, 2 or 3, characterised in that absorbents such as active charcoal or adsorber resins are added to the culture medium.

5. Process according to one of Claims 1, 2, 3 or 4, characterised in that the incubation is carried out at temperatures from 10 to 45°C.

## Revendications

1. Procédé de préparation de vanilline naturelle, caractérisé en ce que l'on procède à une oxydation microbienne d'eugénol et/ou d'iso-eugénol en vanilline à l'aide de microorganismes des genres Serratia, Klebsiella ou Enterobacter.

2. Procédé selon la revendication 1, caractérisé en ce que l'on utilise comme microorganismes des genres Serratia, Klebsiella ou Enterobacter des souches de Serratia ficaria, Serratia fonticola, Serratia liquefaciens, Serratia marcescens, Serratia marinorubra, Serratia odorifera, Serratia plymuthica, Serratia rubidaea, Enterobacter aerogenes, Enterobacter agglomerans, Enterobacter cloacae, Klebsiella aerogenes, Klebsiella pneumoniae ou Klebsiella edwardsii.

3. Procédé selon la revendication 1, caractérisé en ce que l'on effectue l'oxydation microbienne dans des milieux de culture classiques.

4. Procédé selon la revendication 1, 2 ou 3, caractérisé en ce qu'on ajoute au milieu de culture un agent adsorbant tel que du charbon actif ou des résines adsorbantes.

5. Procédé selon l'une des revendications 1, 2, 3 ou 4, caractérisé en ce que l'on effectue l'incubation à des températures de 10 à 45°C.
